Europäisches Patentamt

European Patent Office

Office européen des brevets

(11) Veröffentlichungsnummer: **0 414 987 A1**

# EUROPÄISCHE PATENTANMELDUNG

(21) Anmeldenummer: **90101764.0**

(51) Int. Cl.5: **G01N 33/00**

(22) Anmeldetag: **30.01.90**

(30) Priorität: **30.08.89 DE 3928697**

(43) Veröffentlichungstag der Anmeldung:
**06.03.91 Patentblatt 91/10**

(84) Benannte Vertragsstaaten:
**AT BE CH DE ES FR GB IT LI NL SE**

(71) Anmelder: **AUERGESELLSCHAFT GMBH**
**Thiemannstrasse 1**
**W-1000 Berlin 44(DE)**

(72) Erfinder: **Misera, Herbert A.**
**Hochtristenweg 15**
**W-1000 Berlin 47(DE)**

(54) Fernmesskopf für Gasmessgeräte.

(57) Die Erfindung beschreibt einen Fernmeßkopf für Gasmeßgeräte, mit denen die Konzentration brennbarer Gase und/oder Dämpfe meßbar ist. Für eine Erneuerung von defekten Sensor-Meßelementen, die gegen intakte Meßelemente vor Ort ausgetauscht werden sollen, wird dies dadurch erreicht, daß der Deckel des den Fernmeßkopf (1) aufnehmenden Gehäuses (2) als ein die Sensor-Meßelemente (6) tragendes Gehäuseteil (5) ausgebildet ist. Dieses Gehäuseteil (5) ist an das die Anschlußeinheit (3) aufnehmende Gehäuse (2) auswechselbar angeordnet.

Fig.1

Die Erfindung bezieht sich auf einen Fernmeßkopf für Gasmeßgeräte nach dem Oberbegriff des Anspruchs 1.

Bei einem bekannten Fernmeßkopf der gattungsgemäßen Art, sind die Sensor-Meßelemente in einem Gehäuse vollständig eingekapselt und in einem weiteren Gehäuseteil angeordnet, in dem die Anschlußeinheit zum Anschluß des Fernmeßkabels untergebracht sind. Hierbei ist das Sensorgehäuse mit der die Meßelemente abdeckenden Sinterscheibe an dem hochgezogenen Rand des Fernmeßkopf-Gehäuses angeordnet, wobei die Sinterscheibe aus dem Rand etwas herausragt und somit mit der Außenatmosphäre in Verbindung steht. Für den Fall, daß die Sensor-Meßelemente im Laufe der Zeit verbraucht bzw. defekt werden, mußte bisher das gesamte Fernmeßkopf-Gehäuse mit der Anschlußeinheit und den defekten Sensor-Meßelementen ausgebaut und zur Erneuerung der Sensor-Meßelemente an den Hersteller der Sensor-Meßelemente eingesandt werden. Dies ist offensichtlich ein Nachteil.

Der Erfindung liegt die Aufgabe zugrunde, diesen vorstehend genannten Nachteil bei einem Fernmeßkopf der eingangs genannten Art zu beseitigen, und einen Fernmeßkopf zu schaffen, der bei einer erforderlichen Erneueurung von defekten Sensor-Meßelementen im Meßsystem verbleibt, und die defekten Sensor-Meßelemente gegen intakte Meßelemente vor Ort austauschbar sind.

Diese Aufgabe wird dadurch gelöst, daß der Deckel als ein die Sensor-Meßelemente tragendes Gehäuse-Teil ausgebildet und an dem die Anschlußeinheit aufnehmenden Gehäuse abnehmbar angeordnet ist.

Die mit der Erfindung erzielten Vorteile bestehen insbesondere darin, daß mit einfachen Mitteln defekte Sensor-Meßelemente ohne Ausbau des Fernmeßkopfes aus dem Meßsystem ausgewechselt werden können, und zwar unter Beibehaltung aller elektrischen Anschlüsse innherhalb des Fernmeßsystems.

Weitere vorteilhafte Ausgesaltungen der Erfindung ergeben sich aus den Unteransprüchen 1 bis 5.

Ein Ausführungsbeispiel der Erfindung ist in der Zeichnung dargestellt und wird im folgenden näher beschrieben. Es zeigt

Fig. 1 eine Schnittdarstellung des Fernmeßkopfes, und

Fig. 2 eine Schnittdarstellung des erfindungsgemäßen Gehäuseteils mit Sensor-Meßelementen

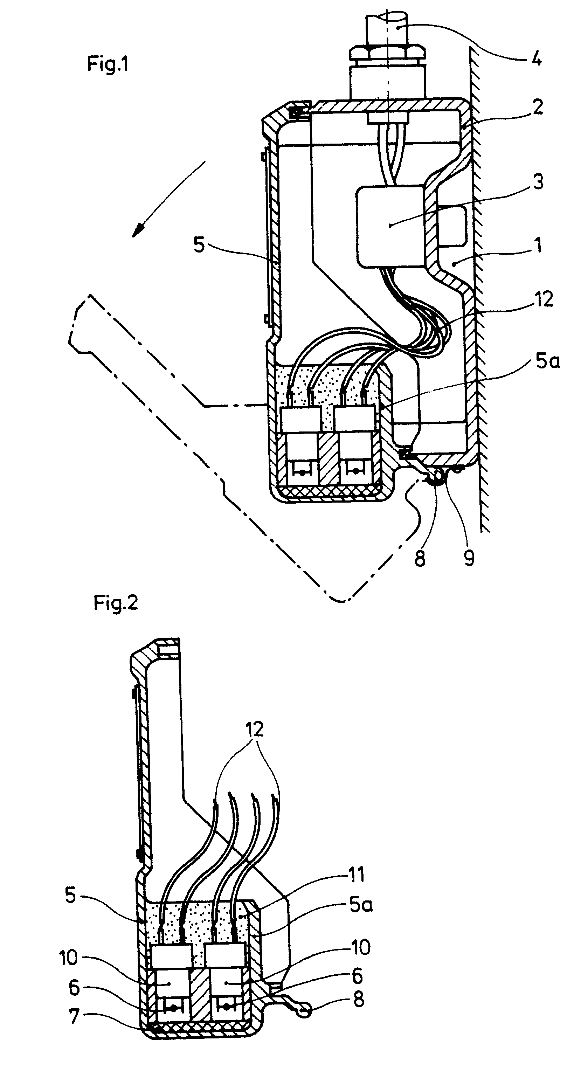

Wie aus Fig. 1 ersichtlich ist, besteht der Fernmeßkopf 1 im wesentlichen aus einem Gehäuse 2 zur Aufnahme einer Anschlußeinheit 3 zum Anschluß eines Fernmeßkabels 4 und aus einem das Gehäuse 2 dicht verschließenden Gehäuseteil 5, in dem die Sensor-Meßelemente 6 und eine die Meßelemente zur Außenatmosphäre hin abdeckende Sinterscheibe 7 oder eine Schutzvorrichtung angeordnet ist. Das Gehäuseteil 5 ist am Gehäuse 2 abnehmbar angeordnet, und zwar mittels eines am Gehäuseteil 5 angeformten Gelenkarms 8, der in ein am Gehäuse 2 entsprechend ausgebildetes Gegenstück 9 eingreift.

Die Fig. 2 zeigt das vom Gehäuse 2 abgenommene Gehäuseteil 5, in dem zur Aufnahme der Sensor-Meßelemente 6 ein topfartiges Formteil 5a ausgebildet ist. Hierbei sind die Sensor-Meßelemente 6 in als Meßkammern ausgebildete Stecker 10 angeordnet, die in das Formteil 5a eingesetzt und mittels einer Vergußmasse 11 im Formteil unverlierbar festgelegt werden. Im Falle eines Defektes der Sensor-Meßelemente 6, wird das Gehäuseteil 5 mit den Meßelementen vom Betreiber der Fernmeßköpfe 1 lediglich gegen ein neues ausgetauscht, wobei dieses neue Gehäuseteil 5 eine vollständig geprüfte und gutachterlich zugelassene Meßelementen-Einheit darstellt. Das Gehäuse 2 mit der Anschlußeinheit 3, verbleibt vorteilhaft mit allen elektrischen Anschlüssen im Fernmeßsystem.

Die Stecker 10 der Sensor-Meßelemente 6, sind mit Anschlußdrähten 12 an die Anschlußeinheit 3 angeschlossen. Beim Auswechseln des Gehäuseteils, sind lediglich diese Anschlußdrähte 12 von der Anschlußeinheit 3 zu lösen.

## Ansprüche

1. Fernmeßkopf für Gasmeßgeräte, mit denen die Konzentration brennbarer Gase und/oder Dämpfe meßbar ist , bestehend aus einem die Sensor-Meßelemente und die Anschlußeinheit zum An-schluß des Fernmeßkabels aufnehmenden Gehäu-se mit abschließendem Deckel, wobei die Meßelemente zur Außenatmosphäre hin von einer porösen Abdeckung abgeschlossen sind, dadurch gekennzeichnet, daß

a) der Deckel als ein die Sensor-Meßelemente (6) tragendes Gehäuseteil (5) ausgebildet ist, und

b) das Gehäuseteil (5) an das die Anschlußeinheit (3) aufnehmende Gehäuse (2) auswechselbar angeordnet ist.

2. Fernmeßkopf nach Anspruch 1, dadurch gekenhzeichent, daß im Gehäuseteil (5) zur Aufnahme der Sensor-Meßelemente (6) ein topfartiges Formteil (5a) ausgebildet ist.

3. Fernmeßkopf nach den Ansprüchen 1 und 2, dadurch gekennzeichnet, daß die Sensor-Meßelemente (6) in als Meßkammern ausgebildete Stecker (10) angeordnet sind, die in das Formteil (5a) eingesetzt und mittels einer Vergußmasse (11) unverlierbar festgelegt sind.

4. Fernmeßkopf nach den Ansprüchen 1 bis 3, dadurch gekennzeichnet, daß das Gehäuseteil (5) als eine auswechselbare Meßelementeneinheit ausgebildet ist.

5. Fernmeßkopf nach den Ansprüchen 1 und 4, dadurch gekennzeichnet, daß am Gehäuseteil (5) ein Gelenkarm (8) ausgebildet ist, der in ein am Gehäuse (2) entsprechend ausgebildetes Gegenstück (9) eingreift.

Fig.1

Fig.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,A | EP-A-0 016 351   (CERBERUS AG.) <br> * Zusammenfassung; Ansprüche 1, 2, 6, 8; Figur 1 * <br> – – – | 1,2-4 | G 01 N 33/00 |
| A | WO-A-8 404 967   (HARALDSTED H.) <br> * Zusammenfassung; Ansprüche 1, 2, 4; Figur 1 * <br> – – – | 1,3,4 | |
| A | FR-A-2 353 846   (AUERGESELLSCHAFT GMBH) <br> * Anspruch 1; Figur 1 * <br> – – – – – | 1,3,4 | |

RECHERCHIERTE SACHGEBIETE (Int. Cl.5)

G 01 N

Der vorliegende Recherchenbericht wurde für alle Patentansprüche erstellt

| Recherchenort | Abschlußdatum der Recherche | Prüfer |
|---|---|---|
| Den Haag | 07 Dezember 90 | BAROCCI S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument
......................................................
& : Mitglied der gleichen Patentfamilie, übereinstimmendes Dokument